# EUROPEAN PATENT APPLICATION

(11) **EP 2 974 751 A1**
(43) Date of publication of application: **20.01.2016**
(21) Application number: 13878105.9
(22) Date of filing: 24.10.2013
(51) Int. Cl.: A61L 27/00, C12N 5/071, C12N 5/077

(54) **CARDIAC OR VASCULAR TISSUE SPHEROID**

(30) Priority: 15.03.2013 JP 2013053037
(71) Applicant: Saga University, Saga-shi, Saga 840-8502 (JP); Cyfuse Biomedical K. K., Bunkyo-ku Tokyo 1130033 (JP)
(72) Inventor: NOGUCHI, Ryo, Saga-shi Saga 840-8502 (JP); TAMURA, Tadashi, Tokyo 113-0033 (JP); NAKAYAMA, Koichi, Saga-shi Saga 840-8502 (JP); MORITA, Shigeki, Saga-shi Saga 840-8502 (JP); NODE, Koichi, Saga-shi Saga 840-8502 (JP)
(74) Representative: Denison, Christopher Marcus
(86) International application number: PCT/JP2013/079478
(87) International publication number: WO 2014/141528

(57) **Abstract**

Provided is a method for producing a cardiac tissue spheroid or vascular tissue spheroid formed from a mixture of a myocardial cell or smooth muscle cell and at least one type of cell selected from a vascular endothelial cell and fibroblast, and a three dimensional cardiac tissue structure or three dimensional vascular tissue structure which are characterized by combining or laminating said spheroid.

## Description

### TECHNICAL FIELD

The present invention relates to a cardiac or vascular tissue spheroid, and a method for producing the same.

### BACKGROUND ART

Recent discoveries of tissue stem cells and the like as well as developments/researches of them for clinical application, such as the iPS cell (reprogramming) technique for which Professor Yamanaka at Kyoto University was awarded the Nobel Prize for Physiology or Medicine in 2012, have been remarkable. While researches on differentiation efficiency, safety, purification and the like aiming at application of the stem cell-derived cells in clinical settings have been going on worldwide, development of techniques for constructing tissues or the like from cells and transplanting them is also considered to be an urgent task.

The cells achieved by these techniques can be utilized in clinical settings, by directly implanting them into the organs or through intravenous or arterial injection. Their poor engraftment efficiency, however, has been considered a problem (Non-Patent Document 1). Meanwhile, an idea of tissue engineering (TE) has been proposed by Vacanti et al. since about 1993, proposing techniques of utilizing biosoluble scaffolds such as collagen and polylactic acid to construct tissues (Non-Patent Document 2). According to this procedure, a foreign substance is used as the scaffold. Therefore, problems such as allergy and infectious diseases against the biosoluble material remain to be solved. Therefore, techniques for producing a tissue with cells alone without a scaffold have been proposed.

As a technique for producing a functional tissue with cells alone, a thermosensitive sheet technique has recently been used in which a sheet that consist of cells alone is formed, which is clinically applied to regenerative medicine intended for cardiac muscle, cornea, esophagus and the like (Non-Patent Document 3). However, techniques for constructing tissues (for example, cardiac muscle, blood vessels, cartilage, valves, etc.) with cells alone such that they have the morphological characteristics and the mechanical action of the tissue have not yet been found and thus regenerative medicine is still thought to be undergoing development.

Meanwhile, as one procedure for forming a tissue that simulates the biological body to a greater extent, a three-dimensional cell formation phenomenon is known in which formation takes place due to aggregation reaction, that is, the nature that adherent cells naturally have. This phenomenon appears by coating a dish or the like with an adhesion inhibitor upon culturing adherent cells, by which the adherent cells aggregate and form a spherical cellular aggregate (referred to as a spheroid). Conventionally reported characteristics of spheroids are advantageous in constructing a three-dimensional tissue as listed below as compared to two-dimensional culture that is conventionally carried out in a culture plate or a flask.

1. Three-dimensional culture allows production of an extracellular matrix in an environment that is closer a biological body.
2. The size of spheroids can be adjusted by adjusting the number of the seeded cells.
3. Handling can be done with a micropipette.
4. Spheroids can be fused with each other.

Spheroids have been used as a tool for analyzing mainly a single cell in cancer research, drug discovery research and tissue engineering (Non-Patent Document 4).

For organ regeneration that allows ideal clinical application, a highly-functional tissue (that functions in the same fashion as a biological body) need to be prepared with cells alone.

### PRIOR ART DOCUMENTS

### Non-Patent Documents

Non-Patent Document 1: Feng Wang, Jianjun Guan, Cellular cardiomyoplasty and cardiac tissue engineering for myocardial therapy, Advanced Drug Delivery Reviews 62 (2010) 784-797
Non-Patent Document 2: Joseph P, Robert Langer, Tissue engineering: the design and fabrication of living replacement devices for surgical reconstruction and transplantation, Molecular medicine 354, 32-34,1999
Non-Patent Document 3: Jun Fujita, Yuji Itabashi, Tomohisa Seki, Myocardial cell sheet therapy and cardiac function. Am J Physiol Heart Circ Physiol 303: H1169-H1182, 2012.
Non-Patent Document 4: Vladimir Mironov, Richard P. Visconti, Vladimir Kasyanov, Organ printing: Tissue spheroids as building blocks, Biomaterials 30 (2009) 2164-2174

### SUMMARY OF THE INVENTION

### Problem to be Solved by the Invention

The present invention aims at providing a method for constructing a cardiac tissue, vessels and the like with cells alone.

### Means for Solving the Problems

The present inventors have gone through intensive studies, as a result of which they succeeded in producing cardiac and vascular tissue spheroids that each simulate the functions in a biological the body by using a mixture of cardiomyocytes, endothelial cells and fibroblasts or a mixture of smooth muscle cells, endothelial cells and fibroblasts, respectively, thereby accomplishing the present invention.

Thus, the present invention is as follows.
(1) A cardiac tissue spheroid formed from a mixture of cardiomyocytes and at least one type of cells selected from vascular endothelial cells and fibroblasts.
(2) The cardiac tissue spheroid according to (1), formed from a mixture of cardiomyocytes, vascular endothelial cells and fibroblasts.
(3) The cardiac tissue spheroid according to (2), wherein the mixture ratio of the cardiomyocytes, the vascular endothelial cells and the fibroblasts is such that the vascular endothelial cells are 10-60 and the fibroblasts are 10-60 to the cardiomyocytes of 100.
(4) A cardiac tissue spheroid obtained by fusing a spheroid formed from cardiomyocytes and a spheroid formed from at least one type of cells selected from vascular endothelial cells and fibroblasts.
(5) The cardiac tissue spheroid according to (4) obtained by fusing a spheroid formed from cardiomyocytes, a spheroid formed from vascular endothelial cells and a spheroid formed from fibroblasts.
(6) The cardiac tissue spheroid according to (5), wherein the abundance ratio of the spheroid derived from cardiomyocytes, the spheroid derived from vascular endothelial cells and the spheroid derived from fibroblasts is such that the spheroid derived from vascular endothelial cells is 10-60 and the spheroid derived from fibroblasts is 10-60 to the spheroid derived from cardiomyocytes of 100.
(7) A method for producing a three-dimensional cardiac tissue structure, comprising the step of compounding or laminating the cardiac tissue spheroids according to any one of (1) to (6) above.
(8) The method according to (7), wherein the step of laminating the spheroids is carried out by using a support comprising a substrate and filamentous bodies or needle-shaped bodies that are arranged substantially vertical to the substrate.
(9) An artificial cardiac tissue comprising the cardiac tissue spheroid according to any one of (1) to (6) above or the three-dimensional structure produced by the method according to either one of (7) and (8).
(10) A vascular tissue spheroid formed from a mixture of smooth muscle cells and at least one type of cells selected from vascular endothelial cells and fibroblasts.
(11) The vascular tissue spheroid according to (10) formed from a mixture of smooth muscle cells, vascular endothelial cells and fibroblasts.
(12) The vascular tissue spheroid according to (11), wherein the mixture ratio of the smooth muscle cells, the vascular endothelial cells and the fibroblasts is such that the vascular endothelial cells are 10-60 and the fibroblasts are 10-300 to the smooth muscle cells of 100.
(13) A vascular tissue spheroid obtained by fusing a spheroid formed from smooth muscle cells and a spheroid formed from at least one type of cells selected from vascular endothelial cells and fibroblasts.
(14) The cardiac tissue spheroid according to (13) obtained by fusing a spheroid formed from smooth muscle cells, a spheroid formed from vascular endothelial cells and a spheroid formed from fibroblasts.
(15) The vascular tissue spheroid according to (14), wherein the abundance ratio of the spheroid derived from smooth muscle cells, the spheroid derived from vascular endothelial cells and the spheroid derived from fibroblasts is such that the spheroid derived from vascular endothelial cells is 10-60 and the spheroid derived from fibroblasts is 10-300 to the spheroid derived from smooth muscle cells of 100.
(16) A method for producing a three-dimensional vascular tissue structure comprising the step of compounding or laminating the vascular tissue spheroids according to any one of (10) to (15) above.
(17) The method according to (16), wherein the step of laminating the spheroids is carried out by using a support comprising a substrate and filamentous bodies or needle-shaped bodies that are arranged substantially vertical to the substrate.
(18) An artificial vascular tissue comprising the vascular tissue spheroid according to any one of (10) to (15) above or the three-dimensional structure produced by the method according to either one of (16) and (17).

### EFFECT OF THE INVENTION

The present invention provides cardiac and vascular tissue spheroids and a method for producing the same. In addition, a three-dimensional cardiac or vascular tissue structure can be produced by compounding or laminating said spheroids. A tissue produced according to the method of the present invention simulates the functions in a biological body and can be used as an artificial cardiac tissue or an artificial vascular tissue. Thus, the present invention is extremely useful in that it can be utilized for regenerative medicine.

### BRIEF DESCRIPTION OF DRAWINGS

[Figure 1] A diagram showing the sizes (unit: µm) of spheroids made from cardiomyocytes alone.
[Figure 2] A diagram showing the relationships between the diameters (µm) and the cell numbers (cells/spheroid) of the spheroids prepared from vascular smooth muscle cells, vascular endothelial cells and fibroblasts, respectively (formation on Day 1).
[Figure 3] A diagram showing the results from studying the relationships between the pulsation efficiency of the cardiac muscle spheroids and the culture solutions.
[Figure 4] A diagram showing the results from studying the relationships between the pulsation efficiency of the cardiac muscle spheroids and the culture solutions.
[Figure 5] Pictures showing formations of spheroids with the respective cells at the indicated mixture ratio.
[Figure 6] Pictures showing spheroids having three-dimensional vascular network.
[Figure 7] Pictures showing spheroids having three-dimensional vascular network.
[Figure 8] A diagram showing the results from analyzing the pulsations of the cardiac tissue spheroids.
[Figure 9] Pictures showing vascular spheroids forming vascular networks and beating.
[Figure 10] Pictures showing fused spheroids that are each formed using one of the three types of cells alone.
[Figure 11] Pictures showing production of a vascular patch tissue.
[Figure 12] Pictures showing a test of grafting the vascular patch tissue into a heart.
[Figure 13] Pictures showing production of a vascular tissue.

### MODES FOR CARRYING OUT THE INVENTION

Hereinafter, the present invention will be described in detail.

The present invention relates to a cardiac tissue spheroid formed from a mixture of cardiomyocytes and at least one type of cells selected from vascular endothelial cells and fibroblasts. The present invention also relates to a vascular tissue spheroid formed from a mixture of smooth muscle cells and at least one type of cells selected from vascular endothelial cells and fibroblasts.

Furthermore, the present invention relates to a method for producing a three-dimensional cardiac tissue structure comprising the steps of: forming a spheroid from a mixture of cardiomyocytes and at least one type of cells selected from vascular endothelial cells and fibroblasts; and compounding or laminating these spheroids. In addition, the present invention is also a method for producing a three-dimensional vascular tissue structure comprising the steps of: forming a spheroid from a mixture of smooth muscle cells and at least one type of cells selected from vascular endothelial cells and fibroblasts; and compounding or laminating these spheroids.

The cardiac tissue spheroid or a three-dimensional structure thereof or the vascular tissue spheroid or a three-dimensional structure thereof produced according to the method of the present invention can be used as an artificial cardiac tissue or an artificial vascular tissue, respectively.

The present inventors took note of the above-described spheroid formation, and developed a technique to form a more three-dimensionally and highly-functional tissue by combining organ-specific cells of interest to give functions that are more like those of a biological body.

According to the present invention, the mainly composing cells are cardiomyocytes or smooth muscle cells. Herein, these mainly composing cells are referred to as the "main cells". According to the present invention, a spheroid or a three-dimensional structure that is formed using cardiomyocytes as the main cells is referred to as a "cardiac tissue spheroid" or a "three-dimensional cardiac tissue structure", respectively. Additionally, according to the present invention, a spheroid or a three-dimensional structure that is formed using smooth muscle cells as the main cells is referred to as a "vascular tissue spheroid" or a "three-dimensional vascular tissue structure", respectively.

According to the present invention, the above-mentioned main cells (cardiomyocytes or smooth muscle cells) are mixed with vascular endothelial cells, fibroblasts or a mixture thereof to prepare a cardiac or vascular tissue spheroid. By compounding or laminating these spheroids by any method, the spheroids will fuse to each other in a three-dimensional way, thereby obtaining a three-dimensional cardiac tissue structure or a three-dimensional vascular tissue structure.

### 1. Formation of cardiac or vascular tissue spheroid (1)

According to the present invention, a cell mixture is prepared by mixing the above-described main cells with vascular endothelial cells and/or fibroblasts to form a spheroid with this mixture. Accordingly, one spheroid contains, as its constituent cells, the main cells as well as vascular endothelial cells and/or fibroblasts.

The mixture ratio of the cells used for preparing a spheroid is as follows.

In a case where a cardiac tissue spheroid is to be prepared using two types of cells, namely, cardiomyocytes and vascular endothelial cells, the vascular endothelial cells are 10-60, preferably 10-30, to the cardiomyocytes of 100.

In a case where a cardiac tissue spheroid is to be prepared using two types of cells, namely, cardiomyocytes and fibroblasts, the fibroblasts are 10-60, preferably 10-30, to the cardiomyocytes of 100.

In a case where a cardiac tissue spheroid is to be prepared using three types of cells, namely, cardiomyocytes, vascular endothelial cells and fibroblasts, the vascular endothelial cells are 10-60, preferably 10-20, while the fibroblasts are 10-60, preferably 10-20, to the cardiomyocytes of 100.

The "fibroblasts" used with the present invention are cells that makes components of dermis such as collagen, elastin and hyaluronic acid. Herein, skin-derived fibroblasts are used.

According to the present invention, besides the vascular endothelial cells and the above-described fibroblasts, other vascular endothelial cells, fibroblasts, mesenchymal stem cells or the like may also be mixed. Examples of such cells include mesenchymal stem cells, fat cells, vascular smooth muscle cells, stem cell-derived vascular endothelial cells, stem cell-derived fibroblasts, stem cell-derived vascular endothelial cells and stem cell-derived mesenchymal stem cells. The mixture ratio in this case may be 5-50, preferably 10-50, to the cardiomyocytes of 100, or they may be mixed such that the cardiomyocytes accounts for 40% or more of the whole mixture. By mixing mesenchymal stem cells, further enhancement of the functions of the spheroid can be expected.

In a case where a vascular tissue spheroid is to be prepared using two types of cells, namely, smooth muscle cells and vascular endothelial cells, the vascular endothelial cells are 10-60, preferably 10-30, to the smooth muscle cells of 100.

In a case where a vascular tissue spheroid is to be prepared using two types of cells, namely, smooth muscle cells and fibroblasts, the fibroblasts are 10-300, preferably 100-300, to the smooth muscle cells of 100.

When a vascular tissue spheroid is prepared using three types of cells, namely, smooth muscle cells, vascular endothelial cells and fibroblasts, the vascular endothelial cells are 10-60, preferably 10-30, while the fibroblasts are 10-300, preferably 100-300, to the smooth muscle cells of 100.

In addition, according to the present invention, as already described above, besides the vascular endothelial cells and the fibroblasts, mesenchymal stem cells, stem cell-derived vascular endothelial cells, stem cell-derived vascular smooth muscle cells, stem cell-derived vascular precursor cells, stem cell-derived fibroblasts or the like may also be mixed. The mixture ratio in this case may be 10-300, preferably 100-300, to the vascular smooth muscle cells of 100, or they may be mixed such that the vascular smooth muscle cells account for 10-90% of the whole vascular structure. By mixing mesenchymal stem cells, further enhancement of the functions of spheroid can be expected.

When the thus-obtained cell mixture is cultured on a water repellent or non-cell-adhesive plate, for example, a Teflon (registered trademark)-coated plate, the cells starts to adhere to each other in search of a scaffold, thereby forming a cell aggregate, namely, a spheroid. The culture time that takes until the spheroid is formed is 6-48 hours, preferably 12-48 hours.

As other spheroid formation methods, IWAKI vessel (EZsphere) (http://atg.ushop.jp/rika/hbin/ez2012.html) or a low-adhesive 10cm- or 6cm-dish from other manufacture can be used.

A culture solution used for forming a spheroid may be a standard culture solution generally used for animal cell culture, for example, Dulbecco's MEM medium (DMEM/High glucose), Dulbecco's MEM/Ham's F12 medium, RPMI-1640 medium or the like, to which a serum may be added. When a spheroid is formed by mixing with vascular endothelial cells, preferably, ECM (endothelial cell medium: Sciencell) is used or vascular endothelial cell growth factor is added to the medium.

### 2. Formation of cardiac or vascular tissue spheroid (2)

According to the present invention, while a mixture of multiple types of cells can be contained in a single spheroid as described above, multiple types of spheroids having different types of cells can be formed such that only a single type of cells exist in a single spheroid.

For example, if a cardiac tissue spheroid is to be prepared, in addition to a spheroid derived from cardiomyocytes, a spheroid derived from vascular endothelial cells and/or a spheroid derived from fibroblasts may be prepared so that they can be fused to each other. Alternatively, if a vascular tissue spheroid is to be prepared, in addition to a spheroid derived from smooth muscle cells, a spheroid derived from vascular endothelial cells and/or a spheroid derived from fibroblasts may be prepared so that they can be fused to each other.

The method for forming the spheroids (vessels and culture conditions, etc.) is the same as described above.

### 3. Construction of three-dimensional cardiac or vascular tissue structure

According to the present invention, the spheroids formed as described above can be compounded or laminated so as to prepare a three-dimensional cardiac or vascular tissue structure.

The method for compounding or laminating spheroids in a three-dimensional manner is not particularly limited. For example, spheroids may be cultured in a tube so that the spheroids can fuse to each other to make a larger spheroid mass.

Alternatively, for example, the method described in WO2008/123614 can be employed to prepare a three-dimensional structure. WO2008/123614 describes a support comprising a substrate and filamentous bodies or needle-shaped bodies for penetrating into a cell mass (spheroids) (hereinafter, simply referred to as a "needle-shaped bodies"). This support can be used to place a spheroid at an arbitrary space. In this support, the needle-shaped bodies are provided substantially vertical to the support so that the spheroids can be skewered or laminated. The spheroids can be placed at any three-dimensional space by controlling the number of the spheroids to be skewered with the respective needle-shaped bodies (the needle-shaped bodies placed at the respective coordinates on the support). Once a structure is formed by fusing/binding the spheroids to each other, the support may be removed so as to obtain a spheroid alone as a three-dimensional cell structure as a whole having any shape. While spheroids are known to fuse to each other when they are left close to each other, the above-described support can be used to control the shape of the structure that is formed through fusion of the spheroids, thereby placing the spheroids at any desired three-dimensional space.

Once the spheroids are placed at any three-dimensional space, tissue of interest can be obtained by culturing for a predetermined time.

When multiple types of spheroids that are each formed from a single type of cells are compounded or laminated with each other, the abundance ratio of the spheroids formed from the respective single-type cells may follow the mixture ratio of the cells where multiple types of cells are already mixed to form the spheroids. Alternatively, the ratio of the total of the single-type spheroids other than the main single-type spheroid may be 10-100 to the main single-type spheroid (cardiomyocytes or vascular smooth muscle cells) of 100.

The cells of the thus-constructed three-dimensional cardiac tissue structure will synchronize and start to beat. This beating structure expresses the gap junctions of the heart in a biological body between the cardiomyocytes, expresses extracellular potential similar to an electrocardiogram of a biological body, and activity is sequentially transmitted between the cells through calcium ion channels in the obtained three-dimensional structure. Thus, the actual functions of a heart are considered to have been reproduced.

On the other hand, the constructed vascular tissue has a tubular structure having an elastic property similar to that of a biological body. Since this configuration can tolerate various mechanical stresses (extension and compression), actual functions of a blood vessel are considered to have been reproduced.

Hence, the cardiac tissue spheroid or a three-dimensional structure thereof of the present invention can be utilized as an artificial cardiac tissue while the vascular tissue spheroid or a three-dimensional structure thereof of the present invention can be utilized as an artificial vascular tissue.

Hereinafter, the present invention will be described more specifically by means of examples. The present invention, however, should not be limited to these examples.

### [Example 1]

### (1) Preparation of cells

### (1-1) Preparation of primary cardiomyocytes (fetal rat ventricular cardiomyocytes)

### (CM) (preplating method)

1 to 3-day-old SD (kud:sd) rat neonate was beheaded before opening the chest to isolate the heart.

The heart was washed with HBSS+/+ (Hank's Balanced Salt Solution, containing Mg, Ca: Sigma Aldrich) cooled to 4°C for three times, and then washed with HBSS-/- (free of Mg, Ca) for three times.

Once the atrium and the great arteries are removed to leave only the ventricle, the ventricle was finely cut with scissors to obtain sections with a size of 1 mm or less. These finely cut ventricular sections were placed in a 0.1% trypsin solution (Wako Pure Chemical Industries) in HBSS-/- and left to stand at 4°C for 6 hours.

Subsequently, 10% FBS (Sigma) and a P/S solution in DMEM (Low Glucose) (Wako Pure Chemical Industries) were added to terminate the reaction of the trypsin.

After removing the solution from the vessel as much as possible, Type 2 collagenase (Sigma) in HBSS-/- (free of Mg, Ca) (final concentration 0.8 mg/ml) was added to the vessel to allow reaction in a thermostatic bath at 37°C for 5 minutes. Then, the supernatant was discarded.

Collagenase was again added to the vessel to allow reaction at 37°C for 15-20 minutes. The supernatant was collected through a cell strainer and precipitated at a rotation speed of 1,000 for 5 minutes. The resulting pellets were resuspended in 10% DMEM and seeded on a 10 mm Type I collagen-coated dish (IWAKI). After 60 minutes, only the supernatant was collected from the dish.

As a result, cardiomyocytes purified to about 90% were obtained at a survival rate of 85.6%-94.7%.

### (1-2) Preparation of vascular endothelial cells (EC)

As vascular endothelial cells, Human Cardiac Microvascular Endothelial Cells (HCMEC) were purchased from Cosmo Bio Co., ltd. (selling source: Sciencell) (http://www.primarycell.com/pdf/6000.pdf).

These cells were cultured in a specialized ECM (Endothelial Cell Medium) (to use the 5th to 9th-passage (P5-9) vascular endothelial cells).

Culture was continued in a 10cm dish (Type I Collagen-Coated Dish: IWAKI) until a target cell number was acquired. 0.05% trypsin was used for the subculture.

### (1-3) Preparation of vascular smooth muscle cells (SMC)

As vascular smooth muscle cells, Human Aortic Smooth Muscle Cells (HASMC) were purchased from Cosmo Bio co., ltd. (selling source: Sciencell) (http://www.primarycell.com/pdf/6110.pdf).

These cells were cultured with 10% FBS (Sigma) and a P/S solution in DMEM (Low Glucose) (Wako Pure Chemical Industries) (to use the P5-10 cells). 0.05% trypsin was used for the subculture.

Culture took place using a 10cm dish, a 15cm dish (NUNC, Corning, etc.) or a five-layered BD Falcon cell culture multi-flask as the culture vessel (http://www.bdj.co.jp/falcon/products/falcon-multi-flask.html).

### (1-4) Preparation of fibroblasts (FB)

As fibroblasts, normal human skin fibroblasts (adult) (NHDF-Ad) were purchased from Takara Bio Inc. (selling source: CMW (LONZA)) (http://catalog.takara-bio.co.jp/product/basicinfo.asp?unitid=U100002705).

These cells were cultured with 10% FBS (Sigma) and P/S solution in DMEM (Low Glucose) (Wako Pure Chemical Industries) to use the P5-10 cells (0.05% trypsin was used for the subculture). As the culture vessel, a 10cm dish, a 15cm dish (NUNC, Corning, etc.) or a five-layered BD Falcon cell culture multi-flask were used (http://www.bdj.co.jp/falcon/products/falcon-multi-flask.html).

### (2) Method for forming spheroid

A requisite amount of the above-described cells were cultured and subjected to trypsin treatment to prepare a cell suspension with a predetermined culture solution. In this case, two or three types of cells were mixed in predetermined proportions to prepare the suspension.

The cardiomyocytes were seeded into a plate immediately after being collected by the above-described cardiac muscle purification method, namely, preplating method (Subsection (1-1) above), thereby forming a spheroid.

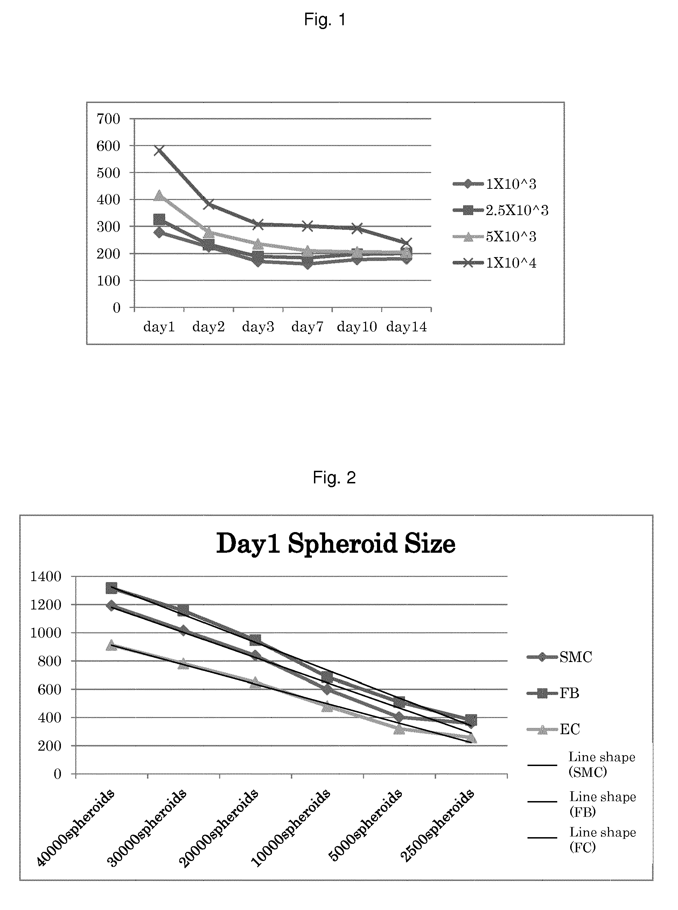

The plate was mainly 96-well plate (prime surface) from Sumitomo Bakelite Co., Ltd., into which a predetermined numbers of the cells were seeded, by which a spheroid was formed 12-48 hours later
(http://www.sumibe.co.jp/product/s-bio/cell-culture/primesurface-96u/spec/index.htm 1). The relationships between the seeded cell numbers and the sizes of the formed spheroid are shown in Figures 1 and 2.

Figure 1 is a diagram showing the cell numbers per spheroid and the diameter of the spheroids that were formed by aggregating the cardiomyocytes alone (unit of the vertical axis: µm).

Figure 2 is a diagram showing the relationships between the diameters and the cell numbers of the spheroids produced from vascular smooth muscle cells, vascular endothelial cells and fibroblasts (formation on Day 1), respectively. For example, "40000 spheroids" of SMC means that a spheroid having a diameter of 1200 (µm) can be obtained when 40000 SMCs are aggregated.

Here, the method for forming a cardiac muscle only spheroid preferably comprises a step of adding about 10% FBS (fetal bovine serum) upon spheroid formation.

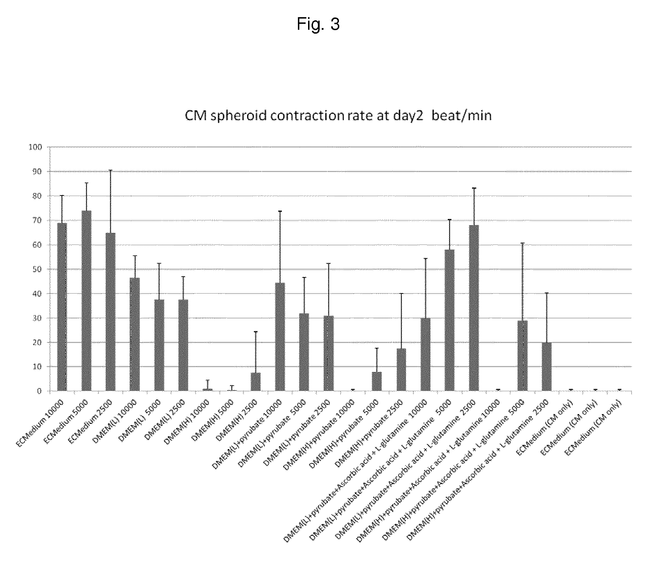

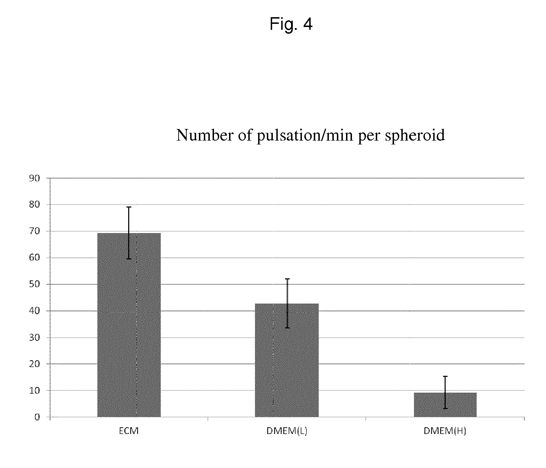

24 to 48 hours after the spheroid was constructed, the cells started to beat. The results from studying the relationship between the pulsation efficiency of the cardiac muscle spheroids and the culture solutions are shown in Figures 3 and 4.

### (3) Various tests of cells

### (3-1) Fluorescent cell tracer

In order to analyze the spheroids during and after the formation thereof, the cells were stained with Cell Tracker.

For the cardiomyocytes, Cell Tracker Green (Takara Bio Inc., (http://catalog.takara-bio.co.jp/product/basicinfo.asp?unitid=U100004642)) was used.

The cells were dissolved in DMEM (FBS-free) at the concentration indicated in the instruction, and stained upon culturing at 37°C under 5% CO₂ environment for 60 minutes by a cardiac muscle purification method, namely, preplating method.

The vascular endothelial cells that reached 80% confluence were washed with PBS, and cultured and stained with Cell Tracker Red (Takara Bio Inc.) dissolved in DMEM (FBS-free) at 37°C under 5% CO₂ environment for 2 hours for use.

The fibroblasts were stained in the same manner using Cell Tracker Blue (Invitorgen)
(http://www.invitrogen.jp/catalogue/molecular_probes/cell_bio_new/content08/index. html).

### (3-2) Microscopic observation

Fluorescence and phase-contrast images of the cells or the three-dimensional structures were captured as still and dynamic images using BZ9000 (KEYENCE).

### (3-3) Dynamic image analysis

VW-9000 (KEYENCE) was used to analyze the functions of the beating cardiac muscle spheroid and structure. Specifically, movements within the spheroid were analyzed at four points (points in the directions of 0, 3, 6 and 9 o'clock) to calculate the average speed.

### (3-4) Histological assessment

The vascular structure was fixed with 10% formalin and subjected to HE staining, MT and EVG staining. The cardiac muscle structure was subjected to HE staining.

### (4) Results

### (4-1) Formation of spheroid

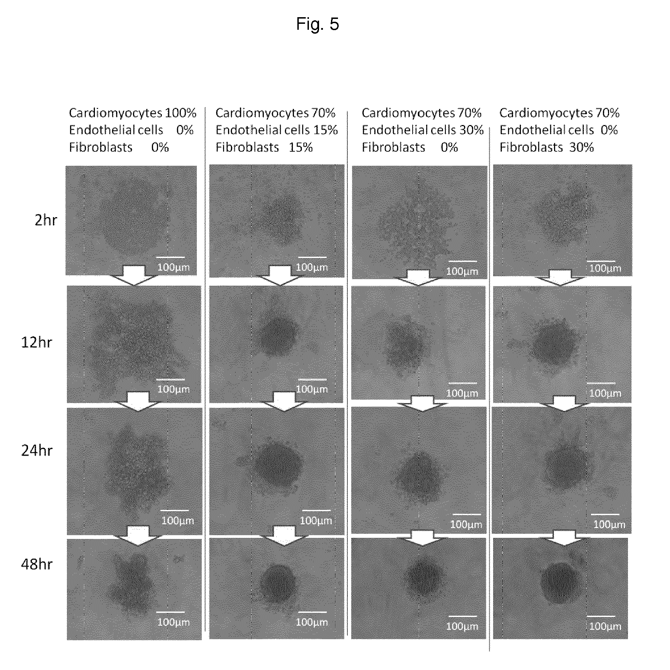

The cardiomyocytes, the vascular endothelial cells and the fibroblasts were mixed in the proportions indicated in Figure 5 to form a cardiac tissue spheroid. The configuration was observed at 2 hours, 12 hours, 24 hours and 48 hours after the formation, to observe the process of how the globular spheroid is formed (Figure 5). Mixing the fibroblasts and the vascular endothelial cells upon forming the spheroid enhanced the efficiency of the spheroid formation.

Three-dimensional functional vascular network was formed with better formation efficiency when spheroids having FB and EC mixed therein were fused than fusing the spheroid of cardiomyocytes alone (Figure 5).

The cell compositions of the spheroids shown in the respective panels in Figure 5 are as follows.
(i) The leftmost panel: Single-cell spheroid containing 100% of cardiomyocytes
(ii) Second panel from the left: Three-type-mixture spheroid containing 80% of cardiomyocytes, 10% of fibroblasts and 10% of vascular endothelial cells.
(iii) Third panel from the left: Two-type-mixture spheroid containing 80% of cardiomyocytes, 0% of fibroblasts and 20% of vascular endothelial cells.
(iv) The rightmost panel: Two-type-mixture spheroid containing 80% of cardiomyocytes, 20% of fibroblasts and 0% of vascular endothelial cells.

In Figure 5, the four panels in the vertical direction respectively represent observation at 2, 12, 24 and 48 hours after seeding the cells for a total cell number of 1200 cells/spheroid to form a spheroid. As compared to the cardiomyocyte-alone group, the spheroids from the mixed cells obviously showed higher aggregation efficiency.

### (4-2) Observation of configuration of spheroid by fluorescent dye staining

The cardiomyocytes, the fibroblasts and the vascular endothelial cells were stained with green, blue and red fluorescent dyes, respectively. They were mixed at a cardiomyocytes: fibroblasts: endothelial cells ratio of 2000: 800: 200 to form spheroids. The configurations were observed 48 hours later.

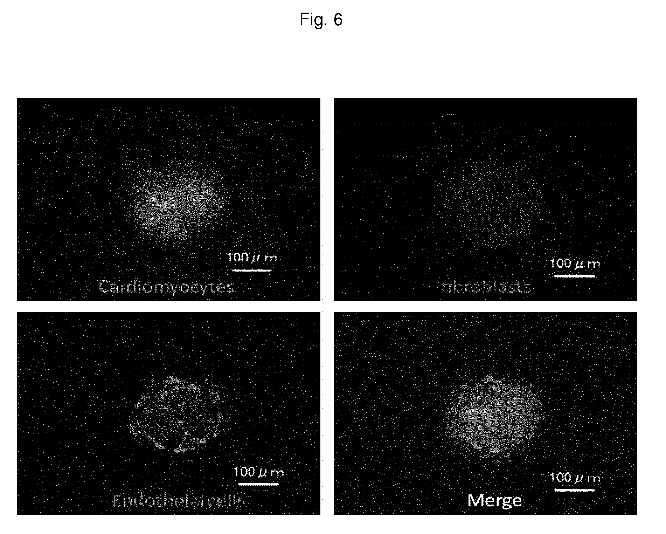

As a result, a highly-functional vascular spheroid assuming a vascular wall was able to be prepared (Figure 6). Moreover, co-culturing the cardiomyocytes with the vascular endothelial cells and the fibroblasts was found to increase the spheroid forming efficiency.

In Figure 6, the vascular endothelial cells formed three-dimensional vascular network where the endothelial cells were arranged in a luminal shape (Figure 6, lower left panel). Specifically, when the cardiomyocytes, the vascular endothelial cells and the fibroblasts are mixed in appropriate compounding proportions, a spheroid having three-dimensional vascular network was found to be prepared.

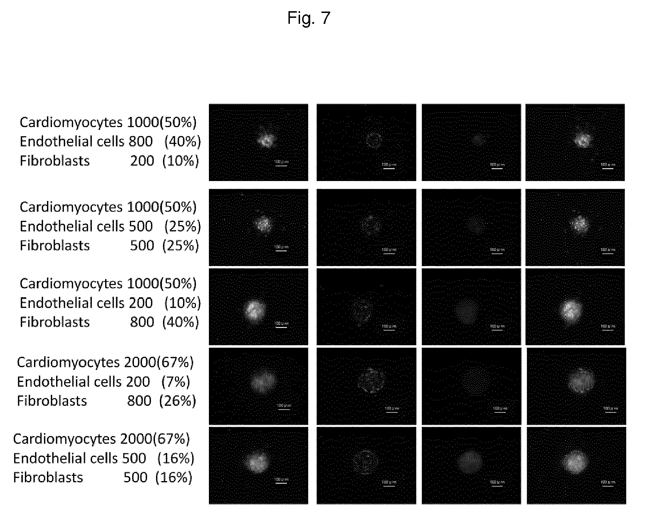

The mixture ratio of the vascular endothelial cells is preferably about 10-50%. If the proportion is increased, dead cells will be increased, which is likely to cause the ultimately formed spheroid to be smaller (Figure 7).

Figure 7 is a diagram showing the results from observing the configuration, the size and the condition of the endothelial cells by mixing the cardiomyocytes, the vascular endothelial cells and the fibroblasts in predetermined compounding proportions and staining them with fluorescent dyes. The compounding proportions of the respective cells are shown on the left side of the respective panels in Figure 7.

Even when the compounding proportion of the vascular endothelial cells was about 10%, vascular network was able to be constructed (Figure 7, third panels from the top). In this case, if the proportion of the fibroblasts is larger than that of the vascular endothelial cells, more three-dimensionally and more complicated vascular network can be formed (Figure 7, fourth panels from the top).

Even if the fibroblasts were mixed at a ratio of as low as about 10%, the efficiency of spheroid aggregation increased. The spheroid forming efficiency improved with the increase in the proportion (Figure 7, uppermost panels). On the other hand, when the proportion of the non-cardiomyocytes was increased to 40% or higher, the pulsation efficiency decreased.

### (4-3) Analysis of pulsation of spheroid

Spheroids were made by compounding the cardiomyocytes at ratios of six levels from 0 to 100% where the non-cardiomyocytes, i.e., the fibroblasts and the vascular endothelial cells, were compounded at 1: 1. The pulsations of these spheroids were recorded for 10 seconds as dynamic images to dynamically analyze the points on the surface of the spheroid in the directions of 0, 3, 6 and 9 o'clock.

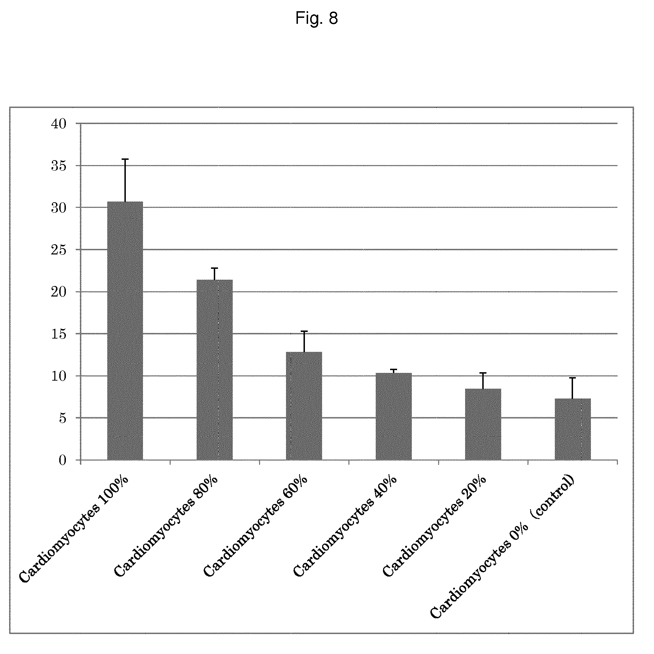

The results are shown in Figure 8. The vertical axis shown in Figure 8 represents the average absolute speed of the moving points. The larger this value is, that is, the higher the average speed is, the higher the number of beats becomes.

In Figure 8, "Cardiomyocytes 100%" means that the constituent cells of the spheroid are cardiomyocytes alone while "Cardiomyocytes 80%" means that the cardiomyocytes account for 80%, the vascular endothelial cells account for 10% and the fibroblasts account for 10%. Similarly, "Cardiomyocytes 60%" means that the cardiomyocytes account for 60%, the vascular endothelial cells account for 20% and the fibroblasts account for 20%; "Cardiomyocytes 40%" means that the cardiomyocytes account for 40%, the vascular endothelial cells account for 30% and the fibroblasts account for 30%; "Cardiomyocytes 20%" means that the cardiomyocytes account for 20%, the vascular endothelial cells account for 40% and the fibroblasts account for 40%; and "Cardiomyocytes 0%" means that the cardiomyocyte accounts for 0%, the vascular endothelial cells account for 50% and the fibroblasts account for 50%.

Referring to Figure 8, preferable compounding ranges of the cells seem to be 60-90% for the cardiomyocytes, 10-50% for the vascular endothelial cells and 10-50% for the fibroblasts (Figure 8).

The higher the cardiac muscle ratio is, the better the pulsation efficiency, with the 100% cardiomyocytes being the highest efficiency. With cardiomyocytes alone, however, the functions would not endure since the engraftment efficiency and the survival rate after grafting will be decreased and the physiologic ability of constructing the vascular network will be reduced. Accordingly, the fibroblasts and the vascular endothelial cells are preferably mixed.

### [Example 2]

According to this example, the spheroids are fused to prepare a three-dimensional structure.

Spheroids were formed from the respective cells in the same manner as in Example 1.

The cardiomyocytes, the vascular endothelial cells and the fibroblasts were stained with green, red and blue fluorescent dyes, respectively, and used to form cardiac tissue spheroids. Furthermore, four spheroids were fused to each other and where observed 3, 12 and 24 hours later.

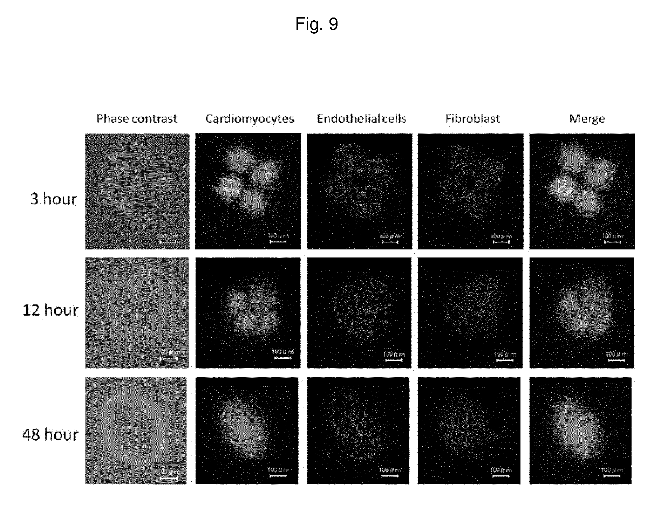

As a result, compounding of the three types of cells formed vascular network and produced a beating cardiac tissue spheroid (Figure 9). As can be appreciated from Figure 9, the three types of cells are found to be fused. In addition, the cells reorganized to some extent and also formed new vascular network.

When these spheroids were fused to each other, they synchronized and started to beat.

### [Example 3]

According to this example, multiple types of spheroids were formed with a single type of cells, respectively. The spheroids were compounded to test the formation of a cardiac tissue.

Spheroids of the respective cells were formed in the same manner as in Example 1.

Three types of spheroids, namely, a spheroid (green) formed from cardiomyocytes alone, a spheroid (red) formed from vascular endothelial cells alone and a spheroid (blue) formed from fibroblasts alone were fused and observed 24 hours later.

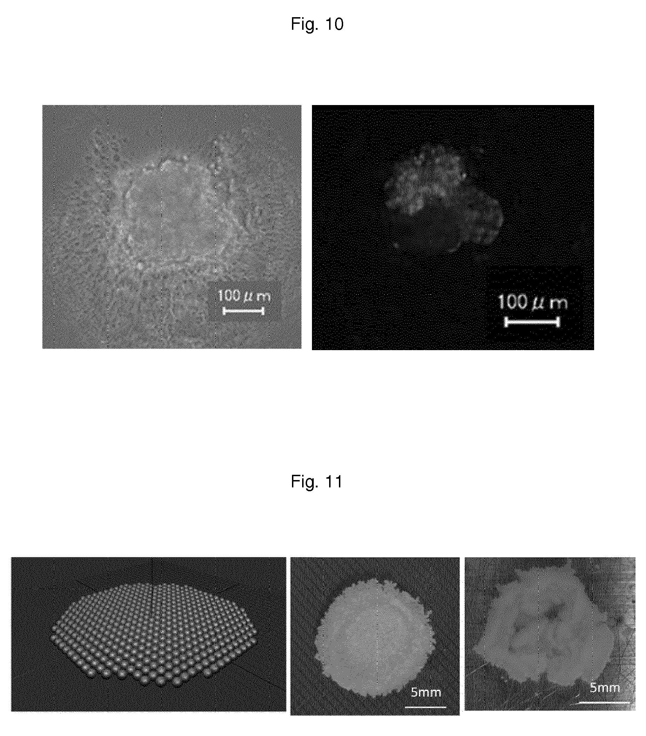

The results are shown in Figure 10. As can be appreciated from Figure 10, three types of spheroids were able to be fused.

### [Example 4]

In this example, a heart vascular tissue was prepared employing the method described in WO2008/123614.

### (1) Preparation of vascular patch

A patch shape shown in the left panel of Figure 11 was designed by computer processing.

About 14,000 spheroids were prepared at about 1000 cells per spheroid, and were fused in an array to have a the patch shape shown in the left panel of Figure 11.

In Figure 11, the middle panel shows patch formation using spheroids formed by mixing vascular smooth muscle cells: fibroblasts: vascular endothelial cells at 3: 2: 1 (2 days after fusion).

The right panel shows similar patch that was formed by forming spheroids with fibroblasts alone (2 days after fusion).

When the patch prepared by using the mixed spheroids of three types of cells (smooth muscle cells, vascular endothelial cells and fibroblasts) was compared with a patch (three-dimensional structure) prepared using spheroids formed with fibroblasts alone, the former obviously had visually smoother surface and thus appropriate as a material as a vascular wall.

### (2) Grafting test of cardiac muscle patch

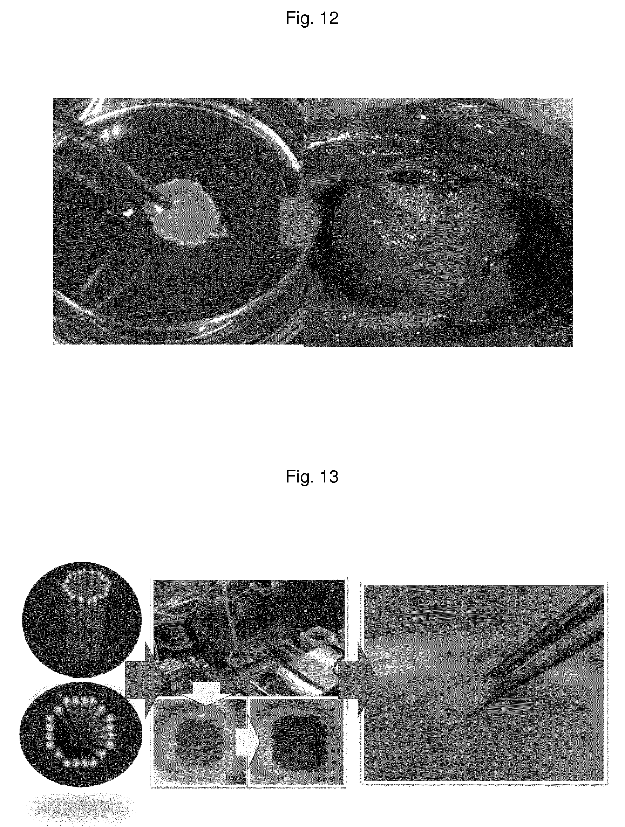

The vascular patch prepared as described above was grafted into the heart of a ischemia-induced F344 nude rat (Figure 12).

### (3) Preparation of vascular tissue

In the same manner as described above, the tubular shape shown in the left panel of Figure 13 was designed and the spheroids were laminated to prepare a vascular tissue structure.

As a result, an elastic defective structure was able to be produced as shown in Figure 13.

Multiple types of cells can be used from biological stem cells, mesenchymal stem cells, fat-derived cells, fat-derived stem cells, embryonic stem cells (ES cells) or induced pluripotent stem cells (iPS cells) harvested upon initialization of differentiated cells.

### INDUSTRIAL APPLICABILITY

The present invention is useful in the following points.
(i) By mixing multiple types of cells, three-dimensional cell structures (heart and vessels) can be prepared with a higher level of functions.
(ii) By fusing spheroids consisting of multiple types of cells, functions and characteristics of a heart or vessels can be provided.
(iii) After forming a spheroid, it may be fused with a cell suspension or a spheroid having different function so as to achieve a higher level of functions.
(iv) By grafting a tissue built from a spheroid or spheroid into a heart, under the skin, blood vessels, muscles, great omentum or the like for engraftment, a therapeutic effect can be achieved.
(iv) A three-dimensional structure prepared according to the method of the present invention can be applied to a drug discovery test or various biological studies.
(v) The method according to the present invention can be applied to iPS cells, ES cells and tissue-stem-cell-derived differentiated cells which are expected of their future clinical applications.

## Claims

1. A cardiac tissue spheroid formed from a mixture of cardiomyocytes and at least one type of cells selected from vascular endothelial cells and fibroblasts.

2. The cardiac tissue spheroid according to Claim 1, formed from a mixture of cardiomyocytes, vascular endothelial cells and fibroblasts.

3. The cardiac tissue spheroid according to Claim 2, wherein the mixture ratio of the cardiomyocytes, the vascular endothelial cells and the fibroblasts is such that the vascular endothelial cells are 10-60 and the fibroblasts are 10-60 to the cardiomyocytes of 100.

4. A cardiac tissue spheroid obtained by fusing a spheroid formed from cardiomyocytes and a spheroid formed from at least one type of cells selected from vascular endothelial cells and fibroblasts.

5. The cardiac tissue spheroid according to Claim 4 obtained by fusing a spheroid formed from cardiomyocytes, a spheroid formed from vascular endothelial cells and a spheroid formed from fibroblasts.

6. The cardiac tissue spheroid according to Claim 5, wherein the abundance ratio of the spheroid derived from cardiomyocytes, the spheroid derived from vascular endothelial cells and the spheroid derived from fibroblasts is such that the spheroid derived from vascular endothelial cells is 10-60 and the spheroid derived from fibroblasts is 10-60 to the spheroid derived from cardiomyocytes of 100.

7. A method for producing a three-dimensional cardiac tissue structure, comprising the step of compounding or laminating the cardiac tissue spheroids according to any one of Claims 1 to 6.

8. The method according to Claim 7, wherein the step of laminating the spheroids is carried out by using a support comprising a substrate and filamentous bodies or needle-shaped bodies that are arranged substantially vertical to the substrate.

9. An artificial cardiac tissue comprising the cardiac tissue spheroid according to any one of Claims 1 to 6 or the three-dimensional structure produced by the method according to either one of Claims 7 and 8.

10. A vascular tissue spheroid formed from a mixture of smooth muscle cells and at least one type of cells selected from vascular endothelial cells and fibroblasts.

11. The vascular tissue spheroid according to Claim 10 formed from a mixture of smooth muscle cells, vascular endothelial cells and fibroblasts.

12. The vascular tissue spheroid according to Claim 11, wherein the mixture ratio of the smooth muscle cells, the vascular endothelial cells and the fibroblasts is such that the vascular endothelial cells are 10-60 and the fibroblasts are 10-300 to the smooth muscle cells of 100.

13. A vascular tissue spheroid obtained by fusing a spheroid formed from smooth muscle cells and a spheroid formed from at least one type of cells selected from vascular endothelial cells and fibroblasts.

14. The cardiac tissue spheroid according to Claim 13 obtained by fusing a spheroid formed from smooth muscle cells, a spheroid formed from vascular endothelial cells and a spheroid formed from fibroblasts.

15. The vascular tissue spheroid according to Claim 14, wherein the abundance ratio of the spheroid derived from smooth muscle cells, the spheroid derived from vascular endothelial cells and the spheroid derived from fibroblasts is such that the spheroid derived from vascular endothelial cells is 10-60 and the spheroid derived from fibroblasts is 10-300 to the spheroid derived from smooth muscle cells of 100.

16. A method for producing a three-dimensional vascular tissue structure comprising the step of compounding or laminating the vascular tissue spheroids according to any one of Claims 10 to 15.

17. The method according to Claim 16, wherein the step of laminating the spheroids is carried out by using a support comprising a substrate and filamentous bodies or needle-shaped bodies that are arranged substantially vertical to the substrate.

18. An artificial vascular tissue comprising the vascular tissue spheroid according to any one of Claims 10 to 15 or the three-dimensional structure produced by the method according to either one of Claims 16 and 17.
